# EUROPEAN PATENT SPECIFICATION

(11) **EP 4 582 121 B1**
(45) Date of publication and mention of the grant of the patent: **15.04.2026**
(21) Application number: 24150304.4
(22) Date of filing: 04.01.2024
(51) Int. Cl.: A61M 11/06

(54) **AEROSOLTHERAPY INSTALLATION COMPRISING A NEBULIZER AND AN AIR-COMPRESSING DEVICE**
AEROSOLTHERAPIEANLAGE MIT ZERSTÄUBER UND LUFTVERDICHTENDER VORRICHTUNG
INSTALLATION DE THÉRAPIE PAR AÉROSOL COMPRENANT UN NÉBULISEUR ET UN DISPOSITIF DE COMPRESSION D'AIR

(43) Date of publication of application: 09.07.2025
(73) Proprietor: Air Liquide Medical Systems S.r.l., 20152 Milano (IT)
(72) Inventor: RUOCCO, Alessandra, 25073 Bovezzo (IT); ALBERICI, Luca, 25073 Bovezzo (IT); SIGNORINI, Alice, 25073 Bovezzo (IT); SANDONI, Giuseppe, 25073 Bovezzo (IT)
(74) Representative: Air Liquide

(56) References cited:
- EP-B1- 3 678 719
- WO-A1-2023/199573
- US-A- 5 054 477

## Description

The present invention concerns an aerosoltherapy installation or equipment useable in aerosoltherapy for aerosolizing or nebulizing a liquid, comprising a nebulizer driven by an air-compressing device, said nebulizer being a portative device designed for being hold in hand by a user, such as a patient in need of an aerosoltherapy treatment.

Nebulizing devices, commonly called nebulizers, are well-known devices useable for delivering an inhalation therapy, i.e. an aerosoltherapy, to a person, typically a patient, in need thereof. Nebulizers are used in combination with a source of compressed gas, typically compressed air, for nebulizing a liquid, typically a drug solution, thereby obtaining a mist or aerosol containing droplets having suitable sizes allowing them reaching the lower airways of the patient that has to inhale said mist in the course of his/her aerosoltherapy.

The source of compressed gas is commonly an air-compressing device comprising a motorized compressing system that sucks, compresses ambient air and provides the thus obtained compressed-air flow to the nebulizer.

Examples of nebulizers are given by EP3912662, EP3459577, EP3721928 and DE199002847, whereas an example of compressing device driven by an electric motor useable for providing compressed air to a nebulizer is given by EP4206467.

The characteristics of the aerosol or mist provided by the nebulizer, such as the droplet or particle size, depend on various features of both the nebulizer and of the compressing device, which are not straightforward to determine. Further, power consumption of the air-compressing device, in particular of the electric motor, may also be an issue from time to time.

For trying to solve those problems, EP3678719 teaches an air-compressing device comprising an electric motor with a stator comprising a laminated core having a thickness of less than 18 mm, with a copper wire winding having a wire diameter of less than 0.35 mm and a number of turns of between 1900 and 2500, that provides compressed air to a nebulizer comprising a compressed-air delivery hole having a diameter greater than 0.55 mm and an ampoule chamber having an inner volume greater than 90 cm³.

However, if such a device works, it has been noticed, in practice, that it may present some drawbacks, such as a power that may be insufficient for effectively sucking the liquid out of the ampoule, especially when the delivery orifice is of a smaller diameter, i.e. of less than 0.55 mm, which may further lead to a velocity and/or a size of the particles forming the aerosol that is/are insufficient, or a concentration or quantity of drug in the mist that is lower than desired.

WO 2023/199573 A1 discloses also an aerosoltherapy installation comprising a nebulizer and an air-compressing device.

A goal of the present invention is to provide an improved aerosoltherapy installation or equipment useable in aerosoltherapy for aerosolizing or nebulizing a liquid, comprising a nebulizer fed with compressed air by an air-compressing device driven by an electric motor, that allows better controlling the droplet velocity, droplet size, and/or concentration and/or quantity of aerosolized drug provided by inhalation to a patient for matching the prescription made by a physician or the like.

A solution according to the present invention concerns an aerosoltherapy installation useable for aerosolizing, i.e. nebulizing, a liquid, such as a drug-containing solution, comprising :
- a nebulizer comprising :
   - a liquid chamber, commonly called an ampoule chamber, having an inner volume of less than 70 cm³ for storing a liquid to be aerosolized, in particular a drug solution, and an inner conduit projecting upwardly in said liquid chamber, said inner conduit comprising an outlet orifice having an orifice diameter of less than 0.55 mm, and
   - an aerosol-generating system comprising a jet nozzle comprising a delivery orifice, and an impactor facing said delivery orifice,
- and an air-compressing device for providing compressed air to the nebulizer, comprising an electric motor comprising a stator having a thickness greater than 18.5 mm, and a wire winding comprising a copper wire having a wire diameter of less than 0.30 mm and a number of turns of between 1900 and 2200.

In relation to the present invention :
- an "aerosol" is a mist containing droplets of liquid, for instance a liquid drug, and a gas, such as air,
- a "gas" is formed of a unique compound (i.e. a pure gas) or of several compounds (i.e. gas mixture) that is/are in gaseous form.
- a "liquid drug" or a "drug-containing solution" is a solution that comprises one or several liquid compounds and optionally one or several solid compounds dissolved, suspended or similar in said one or several liquid compounds.
- the following terms are considered as being strictly equivalent:
   - "aerosol" and "mist",
   - "to aerosolize", "to atomize" and "to nebulize",
   - "compressed air" and "pressurized air",
   - "ampoule chamber", "liquid chamber" and "solution chamber",
   - "drug" and "medication",
   - "liquid" and "solution",
   - "particles" and "droplets",
   - "nebulizer", "nebulizing device" and "nebulization device",
   - "impactor", "pisper" and "deflector".

### Nebulizer

Depending on the embodiment, the nebulizer of the aerosoltherapy installation or equipment according to the present invention can comprise one or several of the following additional features:
- the ampoule chamber, i.e. liquid chamber, has an inner volume of between 30 cm³ and 60 cm³.
- the ampoule chamber has an inner volume of between 40 cm³ and 55 cm³.
- the ampoule chamber has an inner volume of between 44 cm³ and 52 cm³.
- the compressed air outlet has an orifice diameter of between 0,45 mm and 0,54 mm, preferably of between 0,48 mm and 0,52 mm.
- the compressed air outlet has an orifice diameter of about 0,50 mm.
- the inner conduit projecting upwardly in the ampoule chamber is arranged at the center of said ampoule chamber.
- at least a part of the inner conduit projecting upwardly in the ampoule chamber has a (tron)conical shape, preferably at least a portion of its top end comprising the outlet orifice having an orifice diameter of less than 0.55 mm.
- the inner conduit projecting upwardly in the ampoule chamber has an elongated shape.
- the inner conduit projects upwardly in the ampoule chamber along the vertical axis (XX) of the nebulizer.
- the jet nozzle of the aerosol-generating system is arranged as a sleeve around the inner conduit projecting upwardly, while keeping a spacing in-between of preferably less than 2 mm.
- the outlet orifice having an orifice diameter of less than 0.55 mm of the inner conduit projecting upwardly in the ampoule chamber, constitutes a compressed-air exit (i.e. a pressurize-air outlet) of the inner passage (i.e. lumen) of the inner conduit projecting upwardly in the ampoule chamber.
- the outlet orifice of the inner conduit projecting upwardly is arranged to face the delivery orifice of the jet nozzle of the aerosol-generating system, when the jet nozzle of the aerosol-generating system is arranged as a sleeve around the top end of the inner conduit projecting upwardly.
- the outlet orifice of the inner conduit projecting upwardly and the delivery orifice of the jet nozzle are co-axially (i.e. axis XX) arranged.
- the outlet orifice of the inner conduit projecting upwardly provides a flow or jet of pressurized air.
- the inner conduit projecting upwardly in the ampoule chamber further comprises an air inlet, preferably the air inlet is arranged at its bottom end.
- the inner conduit projects upwardly and axially (XX) in the ampoule chamber of the nebulizer.
- the inner conduit comprises a little tube or the like.
- the inner conduit projecting upwardly in the ampoule chamber further comprises an inner passage, i.e. a lumen, fluidly connecting the air inlet to the air outlet for conveying pressurized air from the air inlet to the air outlet.
- the jet nozzle of the aerosol-generating system has a conical or tronconical inner shape.
- the inner conduit of the ampoule chamber comprises an end portion terminated by the air outlet, said end portion comprises an outer shape complementary to the conical or tronconical inner shape of the jet nozzle of the aerosol-generating system.
- the end portion of the inner conduit of the ampoule chamber and the jet nozzle of the aerosol-generating system are coaxially-arranged.
- the inner conduit of the ampoule chamber is in fluid communication with a compressed-air delivery port of the air-compressing device thereby providing compressed air delivered by the air-compressing device to the inner conduit of the nebulizer.
- the impactor is arranged to face the delivery orifice of the jet nozzle so that the jet of compressed-air and liquid sucked out of the ampoule chamber, i.e. inner chamber, impacts the impactor thereby creating the desired mist, i.e. an aerosol.
- the impactor has for instance a blade shape, a wall shape, a ball shape or the like.
- the jet nozzle and the impactor of the aerosol-generating system are made one-piece, for instance molded in one piece.
- the jet nozzle and the impactor are spaced by a distance of less than 3 mm, preferably less than 2 mm.
- the nebulizer further comprises an aerosol chamber for recovering at least a part of the aerosol generated by the aerosol-generating system.
- the aerosol chamber is in fluid communication with a respiratory interface for providing said aerosol to a user.
- the respiratory interface is a mouthpiece or a nasal or facial mask, or the like.
- the respiratory interface is detachably fixed to the nebulizer body and in fluid communication with the nebulization chamber.
- the nebulizer further comprises a nebulizer body comprising the aerosol-generating system and the aerosol chamber, i.e. nebulization chamber.
- according to an embodiment, the ampoule chamber that stores the liquid or solution to be aerosolized is not detachable.
- according to another embodiment, the nebulizer comprises a detachable reservoir comprising at least a part of the ampoule chamber for receiving the liquid or solution to be aerosolized, i.e. the reservoir is detachably fixed to the nebulizer body.
- the nebulizer body comprises a detachable cover.
- the detachable cover forms a top or roof of the nebulization body.
- the cover comprises an elongated tubular element that axially projects in the nebulizer's body.
- the elongated tubular element comprises an upper end and a lower end (i.e. two opposite ends), and an internal passage, i.e. a lumen.
- the internal passage of the elongated tubular element fluidly communicates with the ambient atmosphere via a top aperture located at its upper end.
- the internal passage of the elongated tubular element fluidly communicates with the aerosol chamber via a bottom aperture located at its lower end.
- the impactor is arranged in the internal passage of the elongated tubular element, preferably in the region of its lower end.
- according to another embodiment, the impactor is arranged outside of the internal passage but so as to face the bottom aperture located at the lower end of the elongated tubular element.
- the jet nozzle faces the lower end of the elongated tubular element.
- the jet nozzle comprises a lower rim located close to the bottom of the ampoule chamber, i.e. at a very few distance, for instance less than 2 mm, preferably less than 1 mm so that the liquid, i.e. drug-containing solution, contained, i.e. stored, in said ampoule chamber can pass between said lower rim and said bottom and subsequently travels in the spacing between the jet nozzle of the aerosol-generating system of the nebulizer and the inner conduit projecting upwardly in said ampoule chamber, namely between the inner wall of said jet nozzle and the outer wall of said inner conduit.
- the ampoule chamber has a cup-like shape.
- the inner conduit projects upwardly at the center of the cup-shape ampoule chamber.
- the inner conduit is arranged through the bottom of the ampoule chamber, i.e. it traverses the bottom of the ampoule chamber.
- the compressed air outlet of the inner conduit projecting upwardly in the ampoule chamber is configured for accelerating the air flow provided by the air-compressing device, thereby creating a Venturi or suction effect pulling some liquid or drug solution out of the ampoule chamber.
- once pulled out of the ampoule chamber by said Venturi or suction effect, the liquid mixes with the compressed-air flow delivered by the outlet of the inner conduit projecting upwardly and the resulting air/liquid jet is propelled onto the deflector, i.e. impactor, thereby creating the desired mist or aerosol.
- the mist contains droplets of liquid medication, i.e. liquid drug or dissolved drug particles.
- the nebulizer body has an elongated shape, i.e. along axis XX.
- the nebulizer body has a generally-cylindrical shape.
- the aerosol chamber is arranged above the ampoule chamber in the nebulizer body.
- the nebulizer body comprises a tubular hollow expansion projecting laterally for connecting the respiratory interface thereto.
- the tubular hollow expansion is angled with respect to the vertical axis XX.
- the tubular hollow expansion is in fluid communication with the aerosol chamber.
- the tubular hollow expansion is configured for providing the aerosol or mist contained in the aerosol chamber to the respiratory interface.
- the tubular hollow expansion comprises a tube or the like.
- the tubular hollow expansion comprises a free end for attaching the respiratory interface thereto.
- the jet nozzle and the impactor of the aerosol-generating system and the tubular elongated element are made one-piece, for instance molded in one-piece, preferably of polymer.
- at least a part of the nebulizer, such as the nebulizer body, the ampoule chamber and/or the top cover, is/are made of polymer(s), such as PP, PC, ABS, PA, PSU or any other suitable plastic material.

### Air-compressing device

Depending on the embodiment, the air-compressing device, i.e. also called compressor device, of the aerosoltherapy installation according to the present invention can comprise one or several of the following additional features:
- the electric motor comprises a stator and a rotor.
- the rotor can be made of iron and aluminum.
- the stator and the rotor are arranged in a protective housing, such as a polymer casing or the like.
- the stator comprises a core, preferably a laminated core.
- the wire winding forms at least a part of the core of the stator.
- the laminated core of the stator has a thickness of between 18.5 mm and 25 mm, preferably of less than 24 mm, more preferably of less than 23 mm, more preferably of less than 22 mm.
- the laminated core of the stator has a thickness of between 18.5 mm and 21.5 mm.
- the laminated core of the stator has a thickness of between 19 mm and 21 mm
- the laminated core of the stator has a thickness of about 20 mm.
- the stator comprises a wire winding, preferably a wire winding of copper wire.
- the wire winding of the stator comprises a copper wire having a wire diameter of between 0,25 mm and 0.29 mm, preferably of less than 0,28 mm.
- the wire winding of the stator comprises a copper wire having a wire diameter of at least 0,20 mm, preferably of at least 0,25 mm.
- the wire winding of the stator comprises a copper wire having a wire diameter of between 0,265 mm and 0.275 mm, preferably of of about 0,270 mm.
- the number of turns of the wire winding of the stator is of between 2000 and 2100, preferably of between 2020 and 2080, more preferably of between 2030 and 2070, more preferably of between 2040 and 2060, typically of about 2050.
- it further comprises compressing elements driven by the electric motor for compressing ambient air.
- the compressing elements comprise a compressor member, such as a piston element or the like.
- the compressing elements further comprise a compression chamber.
- the piston element mobile is mobile in the compression chamber, preferably according to a back and forth motion for sucking and compressing air.
- the compressor member, especially the piston element, is -directly or indirectly-fixed to the rotatable shaft of the electric motor.
- the compressing elements further comprise a manifold block for providing air to the compression chamber.
- the air-compressing device is configured for compressing ambient air to a pressure of more than 1 bar abs and/or less than 5 bar abs, preferably of less than 4 bar abs, typically of between 1,2 and 3,5 bar abs.
- the electric motor is powered with electric power, for instance of between 120 and 200 VA.
- it comprises a compressed-air delivery port for providing compressed air to the nebulizer, preferably to the inner conduit the nebulizer.
- the compressed-air delivery port is fluidly connected to the nebulizer by means of a flexible hose or the like.

Embodiments of an aerosoltherapy installation comprising a nebulizer and an air-compressing device according to the present invention are shown in the enclosed Figures, among which:
- Figure 1 represents an embodiment of an aerosoltherapy installation according to the present invention comprising a nebulizer and an air-compressing device.
- Figure 2 represents an embodiment of the motor of the air-compressing device of Fig. 1.
- Figures 3A&3B represent partial sectional views of two embodiments of the nebulizer of Fig. 1.
- Figure 4 shows an embodiment of the ampoule chamber and of an aerosol-generating system useable in the nebulizer of Fig. 1 or of Figures 3A&3B.
- Figure 5 represents a sectional view of the motor of Fig. 2.

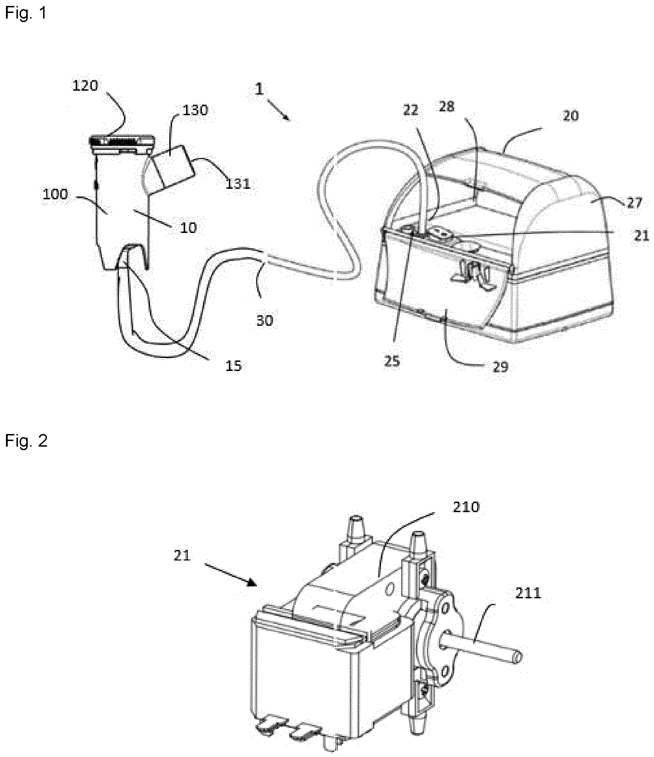

Figure 1 represents shows an aerosoltherapy installation 1 comprising a nebulizer 10 and an air-compressing device 20, also called an air compressor, according to the present invention fluidly connected by a flexible hose 30 or the like that conveys the compressed-air flow provided by the air-compressing device 20 to the nebulizer 10.

The flexible hose 30 that is typically plugged (i.e. at its upstream end) to a compressed-air delivery port 25 arranged on the air compressor 20 and (i.e. at its downstream end) to a compressed-air inlet 15 of the nebulizer 10. The compressed-air delivery port 25 and/or the compressed-air inlet 15 can be arranged on suitable fluidic connectors or the like.

In the nebulizer 10, the compressed-air flow is used for atomizing a liquid, typically a drug-containing solution to be aerosolized, that is subsequently delivered to a patient in need thereof by means of a respiratory interface 19, such as a mouthpiece (as shown) or a nasal or facial mask (not shown), or the like, as detailed below.

The air-compressing device 20 comprises a rigid casing 27 containing in its inner volume 28, compressing elements 22 driven by an electric motor 21 that are used for compressing ambient air (i.e. air at atmospheric pressure) thereby delivering a flow of compressed air, typically at a pressure of less than 5 bar abs, typically of between 1.2 et 3.5 bar abs, or any suitable pressure. A pivotable cover 29 gives access to the inner volume 28 of the casing 27. The air-compressing device 20 further comprises other classical components, such as an on/off button, a power connection to the Mains (110/220 V) or the like...

Compressing elements 22 can comprise an air compression chamber, a compressor element, such as a mobile piston element or the like, driven by the electric motor 21, and preferably a manifold block cooperating together for sucking and compressing ambient air. Ambient air sucked by the compressor element driven by the electric motor 21, passes through the manifold block, then enters into the air compression chamber where it is compressed by the compressor element and, once compressed, exits the air compression chamber and is conveyed to the nebulizer 10, via the compressed air port 25 and the flexible hose 30. Such an arrangement is known and described for instance by EP4206467.

The compressor element, such as a piston element or the like, can be -directly or indirectly- fixed to the rotatable shaft (i.e. axis) 211 of the electric motor 21 as shown in Fig. 2, so as to be, in use, driven by the electric motor 21. The electric motor 21 is powered by a power source (not shown) providing electric current, such as the mains, i.e. 110/220V.

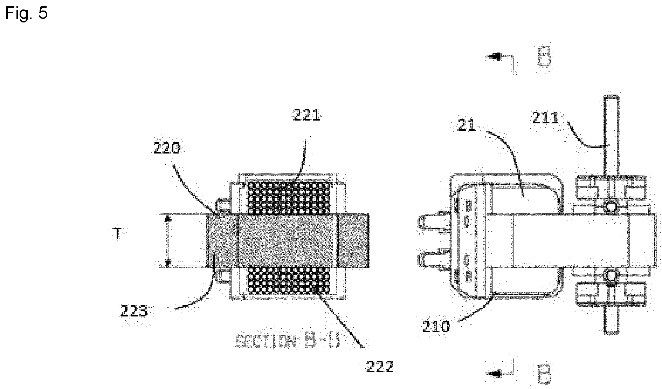

As shown in Figure 5 (sectional view BB), the electric motor 21 further comprises a stator 220 arranged in a rigid housing 210. The stator 220 comprises a wire winding 221 comprising a copper wire 222, i.e. a copper coil or the like.

According to the present invention, the stator 220 of the motor 21 comprises a laminated core 223 having a thickness T greater than 18.5 mm, preferably of between 19 mm and 21 mm, typically of about 20 mm. Further, the wire winding 221 comprises a number of turns of between 1900 and 2200, preferably of between 2000 and 2100 turns, typically of about 2050 turns.

Furthermore, the wire 222 used in said wire winding 221 is a copper wire, i.e. pure copper or a copper alloy, having a wire diameter of less than 0.30 mm, preferably of between 0,25 mm and 0.29 mm, typically of about 0,27 mm.

Combining those features according to the present invention, namely a thickness T greater than 18.5 mm, typically of about 20 mm, a number of turns of between 1900 and 2200, such as of about 2050, and a wire diameter of less than 0.30 mm, typically of about 0,27 mm, leads to an air-compressing device 20 that is more efficient, and exhibits an improved thermal stability but a lower energy consumption.

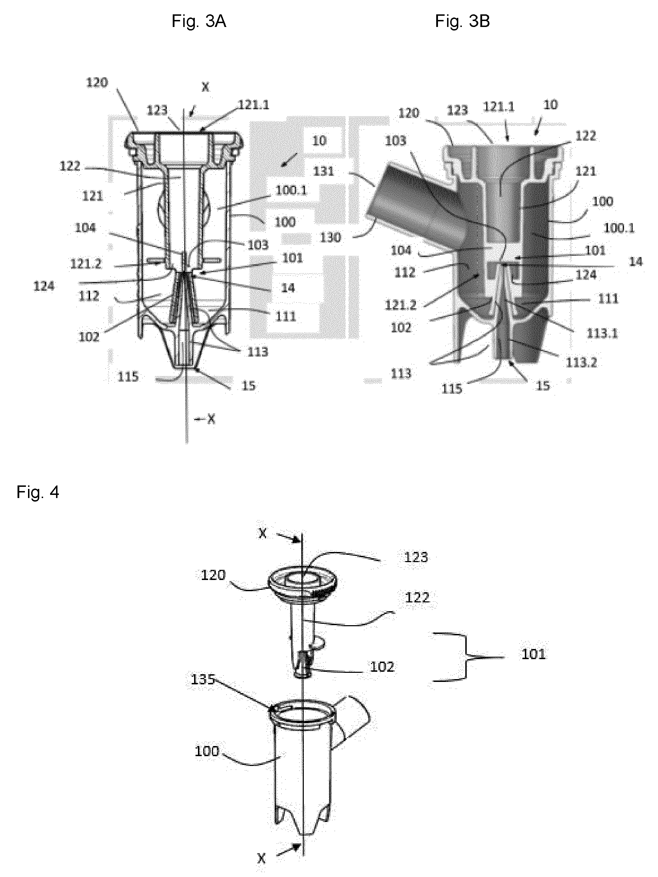

As shown in Figures 3A&3B that represent partial sectional views of two possible embodiments of the nebulizer 10 of the aerosoltherapy installation 1 according to the present invention of Fig. 1.

The nebulizer 10 is preferably made of several parts or sub-units detachably fixed together. For instance, it can comprise as shown in Fig. 3A&3B :
- a main body 100 forming the main part of the nebulizer 10, that comprises the ampoule chamber 111, which is designed for receiving and containing the liquid L to be aerosolized, typically a drug-containing solution, and further an aerosol chamber 112 for recovering the aerosol generated. It has here a generally-cylindrical shape comprising an inner hollow volume 100.1 and a vertical axis XX.
- an aerosol-generating system 101 for generating the desired aerosol, i.e. a mist, which is inserted in the main body 100, i.e. in its inner hollow volume 100.1. The aerosol-generating system 101 comprises an impactor 104 and a jet nozzle 102 as below explained.

The main body 100 is closed by a top cover 120 (cf. Fig. 3A&3B), such as a detachable cover, that forms a top or roof of the nebulization body 100.

In the embodiments of the Figures, the top cover 120 carries an elongated tubular element 121 that axially projects in the nebulizer's body 100, i.e. along the vertical axis XX. The top cover 120 and the elongated tubular element 121 are made one piece as shown in Fig. 4. According to other embodiments, they can be made of two or more sub-units or parts attached together.

The elongated tubular element 121 comprises an upper end 121.1 and a lower end 121.2, namely two opposite ends, and an internal passage 122, i.e. a lumen, that communicates, on the one hand, with the ambient atmosphere via a top aperture 123 located at its upper end 121.1, and, on the other hand, with the aerosol chamber 112 via a bottom aperture 124 located at its lower end 121.2. In other words, the elongated tubular element 121 traverses the top cover 120 at its upper end 121.1.

The elongated tubular element 121 can provide additional ambient air for facilitating the breath of the patient, when the patient inhales. Additional air mixes with the mist created by the aerosol-generating system 101 and recovered in the aerosol chamber 112.

Further, the impactor 104 of the aerosol-generating system 101 is arranged in the internal passage 122 of the elongated tubular element 121, preferably in the region of its lower end 121.2 as shown in Fig. 3A&3B. However, according to other embodiments, the impactor 104 can also be arranged outside of the internal passage 122 but so as to face the bottom aperture 124 located at the lower end 121.2 of the elongated tubular element 121.

As shown in Fig. 3A & 3B, the ampoule chamber 111, i.e. inner chamber, is delimited by peripheral and bottom walls forming a reservoir or tank that receives and stores the liquid to be aerosolized, e.g. a liquid drug. Preferably, the ampoule chamber 111 has a "cup-like" shape.

The ampoule chamber 111 is further traversed by an inner conduit 113, preferably arranged at the center of the ampoule chamber 111, namely at the center of the cup-like" shape. Said inner conduit 113 projects axially (axis XX) and upwardly into the ampoule chamber 111 of the nebulizer 10. As shown in Fig. 3A & 3B, the downstream portion 113.1 of the inner conduit 113 that is located in the ampoule chamber 111, has a conical outer shape, whereas its upstream portion 113.2 has a cylindrical outer shape.

Depending on the embodiment, the ampoule chamber 111 can be arranged in the main body 100 of the nebulizer 10 or be detachable, for instance it can constitute a removable reservoir that is inserted into and detachably fixed to the main body 100 of the nebulizer 10.

The ampoule chamber 111 comprises an inner volume for storing/containing the liquid to be aerosolized, typically a drug-containing solution or the like, that is of less than 70 cm³, preferably of between 30 cm³ and 60 cm³, more preferably of between 40 cm³ and 55 cm³, for instance of about 44.50 cm³ to 51.55 cm³.

Such a reduced volume allows limiting the size of the ampoule chamber 111 thereby better controlling the quantity of liquid drug, without negatively affecting the characteristics of the nebulization.

Furthermore, the inner conduit 113 traversing the ampoule chamber 111 comprises an inner passage 115, i.e. a lumen, in fluid communication with the air inlet 15 of the nebulizer 10, at its upstream end, and with a compressed air outlet 14, at its downstream end. The air outlet 14 of the inner conduit 113 faces the delivery orifice 103 of the jet nozzle 102, preferably they are coaxially (XX) arranged.

The role of the inner conduit 113 is to convey, in its lumen 115, the pressurized air flow provided by the air-compressing device 20 via the flexible hose 30 that is fluidly connected to the air inlet 15 located at the upstream end of the inner conduit 113. The compressed air flow travels into the lumen 115 of the inner conduit 113 and is delivered by the downstream end of the inner conduit 113, through the air outlet 14 and subsequently through the delivery orifice 103 of the jet nozzle 102 for creating the desired mist or aerosol, thanks to the aerosol-generating system 101 that is configured for generating the aerosol, i.e. a mist, with liquid pulled out of the ampoule chamber 111 and compressed air provided by the air-compressing device 20.

The aerosol-generating system 101 comprises a jet nozzle 102 comprising a delivery orifice 103 for delivering a jet of liquid/compressed air toward the impactor 104, also called deflector or pisper, arranged to face the delivery orifice 103 of the jet nozzle 102.

The impactor 104 can have various shapes, for instance it can be or comprise a little wall, a blade, a pin or any other suitable shape. Further, the delivery orifice 103 can have a circular section, an ellipse section or any other suitable shape.

The jet nozzle 102 is arranged as a sleeve around the inner conduit 113 projecting upwardling in the ampoule chamber 111, but at distance from said inner conduit 113. In other words, the jet nozzle 102 is a generally "tubular" shape or the like comprising a preferably conical or tronconical top end carrying the delivery orifice 103, namely a kind of "bell" shape, that matches the shape of the downstream portion 113.1 of the inner conduit 113, i.e. the conical outer shape of the downstream portion 113.1.

A little spacing is kept between the inner wall or surface of the jet nozzle 102 and the outer wall or surface of the inner conduit 113 so that liquid stored in the ampoule chamber 111 can travel in said spacing, when said liquid is sucked out by Venturi effect as below explained. Preferably, the width of the spacing is of less than about 1 mm. As the jet nozzle 102 forms a sleeve around the inner conduit 113, the spacing has an annular section or the like.

Further, the lower end of the jet nozzle 102 projects into the ampoule chamber 111 containing the liquid drug and terminates close to the bottom wall of said ampoule chamber 111, as shown in Figure 3A&3B, preferably at a distance of less than about 2 mm, thereby allowing some liquid to circulates between the surface of the bottom wall of the ampoule chamber 111 and the rim forming the lower end of the jet nozzle 102.

Thus, the liquid contained into the ampoule chamber 111 is able to circulate, i.e. to pass, between the bottom wall of the ampoule chamber 111 and the rim of the lower end of the jet nozzle 102 and then enter and further circulate into the annular spacing, before reaching the aerosol-generating system 101, in particular before impacting the impactor 104, that is configured for generating the aerosol or mist thanks to a Venturi effect.

This principle is well-known in the art. In few words, the compressed air jet is delivered at a high speed and a constant flowrate by the air outlet 14, i.e. a pressurized-air delivery orifice, located at the downstream end of the downstream portion 113.1 of the inner conduit 113 and facing the delivery orifice 103 of the jet nozzle 102. This compressed air jet creates a suction effect that moves, i.e. pulled out, liquid solution out of the ampoule chamber 111. The liquid then travels in the annular spacing, i.e. it is upwardly pulled by the suction effect, as already explained and is then delivered by the delivery orifice 103 of the jet nozzle element 102, toward the deflector or impactor 104, such as a blade or similar, thereby creating the desired mist, i.e. aerosol, that is formed of droplets of liquid, i.e. drug or the like, carried by the air stream.

In other words, when the liquid/gas jet enters into contact with the impactor 104, a mist of liquid droplets is created by its impact on the impactor 104.

The obtained mist or aerosol continues its journey toward the patient, via the aerosol chamber 112 that is located above the ampoule chamber 111 in the main body 100 of the nebulizer 10 as shown in Fig. 3A&B. In other words, the drug droplets forming the aerosol are recovered into the aerosol chamber 112 before being conveyed and delivered to the patient, via a suitable respiratory interface (not shown).

In the embodiments of Fig. 1 and Fig. 3A&B, the nebulizer 10 further comprises a tubular hollow expansion 130 projecting laterally, i.e. a tube, conduit or the like, for connecting the respiratory interface thereto. The tubular hollow expansion 130 is angled with respect to the vertical axis XX, for instance an angle of about 45°. The tubular hollow expansion 130 is further in fluid communication with the aerosol chamber 112 so that the mist contained in the aerosol chamber 112 can be recovered and travel in its lumen toward the patient.

The tubular hollow expansion 130 comprises a free end 131 for attaching the respiratory interface thereto, such as a mouthpiece that the patient can use for inhaling the mist via the mouth.

The aerosol provided by the tubular hollow expansion 130 can thereafter be inhaled by the patient. When a respiratory interface is required, it can be a mouthpiece, or a mouth, nasal or facial respiratory mask, or any other suitable respiratory device. The choice of the most suitable interface depends on the type of medication to be taken and on the type of patients, for instance a facial mask is advocated for pediatric patients, for instance children or toddlers, while a mouthpiece or a mouth mask is more suitable for adults.

The compressed air outlet 14 of the inner conduit 113 should have a precise diameter for ensuring a suitable particle size of the droplets forming the mist as well as an efficient drug amount in the mist. Thus, according to the present invention, the compressed air outlet 14 has an orifice diameter of less than 0.55 mm, preferably of between 0,45 mm and 0,54 mm, more preferably of between 0,48 mm and 0,52 mm, for instance of about 0,50 mm.

The advantages of having such a diameter is to obtained a suitable particle size containing a desired drug content as well as a high particle velocity but a low median mass aerodynamic diameter (MMAD), i.e. a low particle size, that allows the droplets efficiently reaching the lower airways of the patient

In the embodiment shown in Figure 4, the tubular elongated element 122, e.g. a hollow cylinder, the top cap 120 and the aerosol-generating system 101, in particular the impactor (not visible) and the jet nozzle 102 are made one-piece, for instance by injection molding or the like.

The tubular elongated element 122 is axially (axis XX) inserted into and fixed to the main body 100 of the nebulizer 1, via a suitable fixing system 135, for instance by screwing, gluing, interlocking, press-fitting snap-fitting, bayonet system or similar.

Preferably, as shown in Fig. 4, the jet nozzle 102 and the impactor 104 are also made integral with the tubular elongated element 123. For instance, the impactor 104 and/or the jet nozzle 102 can be fixed to the wall of the tubular elongated element 122.

Preferably, the assembly comprising the tubular elongated element 122, the top cap 120, the jet nozzle 102 and the impactor 104 of the aerosol-generating system 101, is removably attached to the main body 100 so that it can be removed for cleaning operations or the like.

In other words, the nebulizer 10 comprises an inner liquid chamber 111, commonly called an ampoule chamber, having a chamber volume for receiving and/or storing the liquid or solution to be nebulized, and further an aerosol-generating system 101 for generating an aerosol or mist. The ampoule chamber 111 has an inner conduit 113 having a conical shape, i.e. its downstream portion 113.1, that terminates with a compressed-air outlet orifice 14 configured for accelerating the air flow provided by the air-compressing device, thereby creating a Venturi or suction effect pulling some liquid or drug solution out of the ampoule chamber 111. The aerosol-generating system 101 comprises a jet nozzle 102 having a delivery orifice 103 for providing a liquid jet comprising liquid sucked out of the ampoule chamber 111. The liquid-containing jet is delivered toward the impactor 104, also called a pisper, a deflector or the like. The desired aerosol or mist is created upon impact of the liquid jet on said impactor 104.

Generally speaking, the different components of the nebulizer 1, i.e. top cover 120, main body 100 comprising the ampoule chamber 111..., are preferably made of plastic material(s), such as PP, PC, ABS, PA, PSU or similar materials, while the respiratory interface, when required, can be made of a soft elastomeric material, such as silicone or the like.

The installation 1 of the present invention is usable for nebulizing a liquid or solution, typically a drug- or medication-containing solution, and thus obtaining a mist or aerosol containing droplets of medication having suitable sizes so as to be able to reach the lower airways of the patient(s) that has/have to inhale said mist in the course of their aerosoltherapy.

## Claims

1. Aerosoltherapy installation (1) useable for aerosolizing, i.e. nebulizing, a liquid comprising :
- a nebulizer (10) comprising :
. a liquid chamber (111) having an inner volume of less than 70 cm³ for storing a liquid to be aerosolized, and comprising an inner conduit (113) projecting upwardly in said liquid chamber (111), said inner conduit (113) comprising an outlet orifice (14) having an orifice diameter of less than 0.55 mm, and
. an aerosol-generating system (101) comprising a jet nozzle (102) comprising a delivery orifice (103), and an impactor (104) facing said delivery orifice (103),
- and an air-compressing device (20) for providing compressed air to the nebulizer (10), comprising an electric motor (21) comprising a stator (220) having a thickness (T) greater than 18.5 mm, and a wire winding (221) comprising a copper wire (222) having a wire diameter of less than 0.30 mm and a number of turns of between 1900 and 2200.

2. Installation according to Claim 1, wherein the liquid chamber (111) of the nebulizer (10) has an inner volume of between 30 cm³ and 60 cm³.

3. Installation according to any one of Claims 1 or 2, wherein the liquid chamber (111) has an inner volume of between 40 cm³ and 55 cm³.

4. Installation according to Claim 1, wherein the outlet orifice (14) of the inner conduit (113) has an orifice diameter of between 0,45 mm and 0,54 mm, preferably of between 0,48 mm and 0,52 mm.

5. Installation according to Claim 1, wherein the core of the stator (220) of the electric motor (21) of the air-compressing device (20) has a thickness (T) of between 18.5 mm and 25 mm, preferably of less than 23 mm.

6. Installation according to any one of Claims 1 or 5, wherein the core of the stator (220) of the electric motor (21) has a thickness of between 18.5 mm and 21.5 mm, typically of about 20 mm.

7. Installation according to any one of Claims 1, 5 or 6, wherein the stator (220) of the electric motor (21) comprises a laminated core.

8. Installation according to Claim 1, wherein the wire winding (221) comprises a copper wire having a wire diameter of between 0,25 mm and 0.29 mm, typically of about 0,27 mm.

9. Installation according to any one of Claims 1 or 8, wherein the number of turns of the wire winding (221) of the electric motor (21) is of between 2000 and 2100, typically of about 2050.

10. Installation according to Claim 1, wherein, in the nebulizer (10):
- the inner conduit (113) projects upwardly at the center of the ampoule chamber (111), and/or
- the jet nozzle (102) is arranged as a sleeve around the inner conduit (113) while keeping a spacing in-between, preferably a spacing of less than 1 mm.

11. Installation according to Claim 1, wherein the inner conduit (113) of the liquid chamber (111) is in fluid communication with a compressed-air delivery port (25) of the air-compressing device (20) thereby providing compressed air to the inner conduit (113) of the nebulizer (10).

12. Installation according to Claim 1, wherein the impactor (104) is arranged to face the delivery orifice (103) of the jet nozzle (102).

13. Installation according to Claim 1, wherein the nebulizer (10) further comprises an aerosol chamber (112) for recovering at least a part of the aerosol generated by the aerosol-generating system (101).

14. Installation according to Claims 1 and 13, wherein the nebulizer (10) further comprises a tubular hollow expansion (130) projecting laterally and in fluid communication with the aerosol chamber (112), preferably the tubular hollow expansion (130) is angled with respect to the vertical axis (XX).

15. Installation according to Claim 1, wherein the nebulizer (10) further comprises a top cap (120) carrying **a** tubular elongated element (123), preferably the top cap (120), the jet nozzle (102), the impactor (104) and the tubular elongated element (123) are made **of** one-piece.

## Patentansprüche

1. Aerosoltherapievorrichtung (1) zur Verwendung bei der Aerosolisierung, d. h. Zerstäubung, einer Flüssigkeit, umfassend:
- einen Zerstäuber (10), der umfasst:
. eine Flüssigkeitskammer (111) mit einem Innenvolumen von weniger als 70 cm³ zur Aufnahme einer zu aerosolisierenden Flüssigkeit, die eine nach oben ragende Innenleitung (113) in der Flüssigkeitskammer (111) umfasst, wobei die Innenleitung (113) eine Auslassöffnung (14) mit einem Öffnungsdurchmesser von weniger als 0,55 mm umfasst, und
. ein Aerosolerzeugungssystem (101) mit einer Strahldüse (102), die eine Auslassöffnung (103) aufweist, und einem Prallkörper (104), der der Auslassöffnung (103) gegenüberliegt,
- und eine Luftkompressionsvorrichtung (20) zum Zuführen von Druckluft zu dem Zerstäuber (10) umfasst, mit einem Elektromotor (21), der einen Stator (220) mit einer Dicke (T) von mehr als 18,5 mm und eine Drahtwicklung (221) mit einem Kupferdraht (222) mit einem Drahtdurchmesser von weniger als 0,30 mm und einer Anzahl von Windungen zwischen 1900 und 2200 umfasst.

2. Aerosoltherapievorrichtung nach Anspruch 1, wobei die Flüssigkeitskammer (111) des Verneblers (10) ein Innenvolumen zwischen 30 cm³ und 60 cm³ aufweist.

3. Aerosoltherapievorrichtung gemäß einem der Ansprüche 1 oder 2, wobei die Flüssigkeitskammer (111) ein Innenvolumen zwischen 40 cm³ und 55 cm³ aufweist.

4. Aerosoltherapievorrichtung gemäß Anspruch 1, wobei die Auslassöffnung (14) der inneren Leitung (113) einen Öffnungsdurchmesser zwischen 0,45 mm und 0,54 mm, vorzugsweise zwischen 0,48 mm und 0,52 mm, aufweist.

5. Aerosoltherapievorrichtung nach Anspruch 1, wobei der Kern des Stators (220) des Elektromotors (21) der Luftkompressionsvorrichtung (20) eine Dicke (T) zwischen 18,5 mm und 25 mm, vorzugsweise weniger als 23 mm, aufweist.

6. Aerosoltherapievorrichtung nach einem der Ansprüche 1 oder 5, wobei der Kern des Stators (220) des Elektromotors (21) eine Dicke zwischen 18,5 mm und 21,5 mm, typischerweise etwa 20 mm, aufweist.

7. Aerosoltherapievorrichtung nach einem der Ansprüche 1, 5 oder 6, wobei der Stator (220) des Elektromotors (21) einen laminierten Kern umfasst.

8. Aerosoltherapievorrichtung nach Anspruch 1, wobei die Drahtwicklung (221) einen Kupferdraht mit einem Drahtdurchmesser zwischen 0,25 mm und 0,29 mm, typischerweise etwa 0,27 mm, umfasst.

9. Aerosoltherapievorrichtung nach einem der Ansprüche 1 oder 8, wobei die Anzahl der Windungen der Drahtwicklung (221) des Elektromotors (21) zwischen 2000 und 2100 liegt, typischerweise bei etwa 2050.

10. Aerosoltherapievorrichtung gemäß Anspruch 1, wobei im Vernebler (10):
- die innere Leitung (113) in der Mitte der Ampullenkammer (111) nach oben ragt und/oder
- die Düse (102) als Hülse um die innere Leitung (113) herum angeordnet ist, wobei ein Abstand dazwischen, vorzugsweise ein Abstand von weniger als 1 mm, eingehalten wird.

11. Aerosoltherapievorrichtung gemäß Anspruch 1, wobei die innere Leitung (113) der Ampullenkammer (111) in Fluidverbindung mit einem Druckluftzufuhranschluss (25) der Luftkompressionsvorrichtung (20) steht, wodurch Druckluft zur inneren Leitung (112) des Zerstäubers (10) geleitet wird.

12. Aerosoltherapievorrichtung gemäß Anspruch 1, wobei der Prallkörper (104) so angeordnet ist, dass er der Auslassöffnung (103) der Strahldüse (102) zugewandt ist.

13. Aerosoltherapievorrichtung gemäß Anspruch 1, wobei der Zerstäuber (10) ferner eine Aerosolkammer (112) zum Rückgewinnen zumindest eines Teils des vom Aerosolerzeugungssystem (101) erzeugten Aerosols umfasst.

14. Aerosoltherapievorrichtung nach den Ansprüchen 1 und 13, wobei der Zerstäuber (10) ferner eine seitlich vorstehende, mit der Aerosolkammer (112) in Fluidverbindung stehende röhrenförmige Hohlverlängerung (130) umfasst, wobei die röhrenförmige Hohlverlängerung (130) vorzugsweise in Bezug auf die vertikale Achse (XX) abgewinkelt ist.

15. Aerosoltherapievorrichtung nach Anspruch 1, wobei der Zerstäuber (10) ferner eine obere Kappe (120) umfasst, die ein röhrenförmiges längliches Element (123) trägt, wobei vorzugsweise die obere Kappe (120), die Strahldüse (102), der Prallkörper (104) und das röhrenförmige längliche Element (123) aus einem Stück hergestellt sind.

## Revendications

1. Installation d'aérosolthérapie (1) utilisable pour aérosoliser, c'est-à-dire nébuliser, un liquide, comprenant :
- un nébuliseur (10) comprenant :
. une chambre à liquide (111) ayant un volume intérieur inférieur à 70 cm³ pour stocker un liquide à aérosoliser, et comprenant un conduit intérieur (113) faisant saillie vers le haut dans ladite chambre à liquide (111), ledit conduit intérieur (113) comprenant un orifice de sortie (14) ayant un diamètre d'orifice inférieur à 0,55 mm, et
. un système de génération d'aérosol (101) comprenant une buse à jet (102) comprenant un orifice de distribution (103), et un impacteur (104) faisant face audit orifice de distribution (103),
- et un dispositif de compression d'air (20) pour fournir de l'air comprimé au nébuliseur (10), comprenant un moteur électrique (21) comprenant un stator (220) ayant une épaisseur (T) supérieure à 18,5 mm, et un enroulement de fil (221) comprenant un fil de cuivre (222) ayant un diamètre de fil inférieur à 0,30 mm et un nombre de tours compris entre 1900 et 2200.

2. Installation selon la revendication 1, dans laquelle la chambre à liquide (111) du nébuliseur (10) a un volume intérieur compris entre 30 cm³ et 60 cm³.

3. Installation selon l'une quelconque des revendications 1 ou 2, dans laquelle la chambre à liquide (111) a un volume intérieur compris entre 40 cm³ et 55 cm³.

4. Installation selon la revendication 1, dans laquelle l'orifice de sortie (14) du conduit interne (113) a un diamètre compris entre 0,45 mm et 0,54 mm, de préférence entre 0,48 mm et 0,52 mm.

5. Installation selon la revendication 1, dans laquelle le noyau du stator (220) du moteur électrique (21) du dispositif de compression d'air (20) a une épaisseur (T) comprise entre 18,5 mm et 25 mm, de préférence inférieure à 23 mm.

6. Installation selon l'une quelconque des revendications 1 ou 5, dans laquelle le noyau du stator (220) du moteur électrique (21) a une épaisseur comprise entre 18,5 mm et 21,5 mm, typiquement d'environ 20 mm.

7. Installation selon l'une quelconque des revendications 1, 5 ou 6, dans laquelle le stator (220) du moteur électrique (21) comprend un noyau laminé.

8. Installation selon la revendication 1, dans laquelle l'enroulement de fil (221) comprend un fil de cuivre ayant un diamètre de fil compris entre 0,25 mm et 0,29 mm, typiquement d'environ 0,27 mm.

9. Installation selon l'une quelconque des revendications 1 ou 8, dans laquelle le nombre de tours de l'enroulement de fil (221) du moteur électrique (21) est compris entre 2000 et 2100, typiquement d'environ 2050.

10. Installation selon la revendication 1, dans laquelle, dans le nébuliseur (10):
- le conduit interne (113) fait saillie vers le haut au centre de la chambre à ampoule (111), et/ou
- la buse à jet (102) est agencée sous la forme d'un manchon autour du conduit interne (113) tout en conservant un espacement entre eux, de préférence un espacement inférieur à 1 mm.

11. Installation selon la revendication 1, dans laquelle le conduit interne (113) de la chambre à ampoule (111) est en communication fluidique avec un orifice d'alimentation en air comprimé (25) du dispositif de compression d'air (20), fournissant ainsi de l'air comprimé au conduit interne (112) du nébuliseur (10).

12. Installation selon la revendication 1, dans laquelle l'impacteur (104) est disposé de manière à faire face à l'orifice de distribution (103) de la buse à jet (102).

13. Installation selon la revendication 1, dans laquelle le nébuliseur (10) comprend en outre une chambre d'aérosol (112) pour récupérer au moins une partie de l'aérosol généré par le système de génération d'aérosol (101).

14. Installation selon les revendications 1 et 13, dans laquelle le nébuliseur (10) comprend en outre une extension tubulaire creuse (130) faisant saillie latéralement et en communication fluidique avec la chambre à aérosol (112), de préférence l'extension tubulaire creuse (130) est inclinée par rapport à l'axe vertical (XX).

15. Installation selon la revendication 1, dans laquelle le nébuliseur (10) comprend en outre un capuchon supérieur (120) portant un élément tubulaire allongé (123), de préférence le capuchon supérieur (120), la buse à jet (102), l'impacteur (104) et l'élément tubulaire allongé (123) sont réalisés d'une seule pièce.
